# EUROPEAN PATENT SPECIFICATION

(11) **EP 3 441 098 B1**
(45) Date of publication and mention of the grant of the patent: **30.09.2020**
(21) Application number: 17185225.4
(22) Date of filing: 08.08.2017
(51) Int. Cl.: A61M 11/06

(54) **NEBULIZER FOR AEROSOLTHERAPY MADE OF SUB-UNITS DETACHABLY SECURED TOGETHER**
VERNEBLER ZUR AEROSOLTHERAPIE AUS LÖSBAR MITEINANDER VERBUNDENEN UNTEREINHEITEN
ATOMISEUR POUR AÉROSOLTHÉRAPIE CONSTITUÉ DE SOUS-UNITÉS FIXÉES ENSEMBLE DE MANIÈRE AMOVIBLE

(43) Date of publication of application: 13.02.2019
(73) Proprietor: Air Liquide Medical Systems S.r.l., 20148 Milano (IT)
(72) Inventor: ALBERICI, Luca, 25030 RONCADELLE (BS) (IT); BUGATTI, Ottorino, 25068 Sarezzo (Bs) (IT); SANDONI, Giuseppe, 25124 BRESCIA (IT)
(74) Representative: Pittis, Olivier

(56) References cited:
- EP-A1- 0 626 180
- EP-A2- 0 855 224
- WO-A1-03/053500
- DE-C1- 19 902 847
- GB-A- 2 358 356
- US-A1- 2011 253 134

## Description

The present invention concerns a nebulizer useable in aerosoltherapy for aerosolizing a liquid, which is made of 4 sub-units or sub-parts that are detachably secured together. The nebulizer of the present invention is a portative device, i.e. a device designed for being hold in hand by a user, i.e. a patient.

Nebulizing devices, commonly called nebulizers, are devices useable for delivering an inhalation therapy, i.e. an aerosoltherapy, to a patient in need thereof. Nebulizers are used in combination with a source of gas, typically compressed air, for nebulizing a drug solution thereby obtaining a mist containing droplets having suitable sizes allowing them reaching the lower airways of the patient that has to inhale said mist in the course of his/her aerosoltherapy.

So far, different architectures of nebulizers have been proposed. However, most of the current nebulizers are not suitable because many of them have a complicated architecture that renders them difficult to assemble/desassemble by the users, i.e. the patients or the medical staff.

Further, most of them comprise a permanent opening for venting the CO₂-containing gases exhaled by the patient during the expiration phases. However, the drawback of such a permanent opening is that the drug-containing mist can also escape through said opening, thereby wasting a significant amount of drug in the environment, i.e. in the surrounding atmosphere.

Nebulising devices are disclosed for example in DE19902847 C1, WO03/053500 A1, GB2358356A, US2011 /253134 A1 and EP0626180 A1, wherein document EP0626180 A1 discloses a device having features as in the preamble of the appended claim 1.

A goal of the present invention is to propose an improved nebulizer that limits the waste of drug in the surrounding atmosphere and that is easy to assemble/disassemble.

A solution according to the present invention is a nebulizer as defined in the appended independent claim 1.

The nebulizer of the present invention has a very intuitive and straightforward architecture as it is made of only 4 parts or sub-units, so that it can be easily assembled or disassembled by the user, and further the presence of the double valve system arranged on the detachable cover limits the waste of liquid drug.

In relation to the present invention :
- an "aerosol" is a mist containing droplets of liquid, for instance a liquid drug, and a gas, such as air,
- a "gas" is formed of a unique compound (i.e. a pure gas) or of several compounds (i.e. gas mixture) that is/are in gaseous form.
- a "liquid drug" or a "drug-containing solution" is a solution that comprises one or several liquid compounds and optionally one or several solid compounds dissolved, suspended or similar in said one or several liquid compounds.

Depending on the embodiment, a nebulizer according to the present invention can comprise one or several of the following additional features:
- the cover comprises a one-way inspiration valve allowing air entering into the nebulization chamber and a one-way expiration valve allowing gas exiting the nebulization chamber.
- the one-way inspiration valve of the cover is configured for allowing gas, such as ambient air, entering into the nebulization chamber of the nebulization body, during the inspiration phases of the patient, but for preventing any exit/escape of gas through said one-way inspiration valve during the expiration phases of the patient.
- the one-way expiration valve is configured for allowing gas, especially CO₂-containing gas, exiting the nebulization chamber during the expiration phases of the patient, but for preventing any entry of gas through said one-way expiration valve during the inspiration phases of the patient.
- the nebulizer body is sandwiched between the cover and the reservoir.
- the nebulizer further comprises a respiratory interface comprising an inner passage, i.e. a lumen, in fluid communication with the nebulization chamber.
- the one-way expiration valve is located between the one-way inspiration valve and the respiratory interface, when the cover is fixed to the nebulizer body.
- the respiratory interface comprises a mouthpiece, a mouth mask or a facial mask.
- the reservoir comprises a liquid tank for receiving a liquid to be aerosolized,
- the aerosol-generating system of the nebulizer body comprises a nozzle element comprising an orifice and a deflector element facing the orifice of the nozzle element.
- the nebulizer body further comprises an axially-arranged inner conduit comprising a lower end projecting into the liquid tank of the reservoir.
- the lower end of the inner conduit terminates close to the bottom of the liquid tank, i.e. at a very few distance, for instance less than 2 mm, preferably less than 1 mm.
- the reservoir further comprises an inner conduit projecting axially and upwardly into the liquid tank.
- the axially-arranged inner conduit of the nebulizer body is arranged as a sleeve around a downstream end of the inner conduit of the reservoir.
- the axially-arranged inner conduit of the nebulizer body is spaced from the downstream end of the inner conduit of the reservoir by a spacing.
- the nozzle element and the deflector element of the aerosol-generating system are arranged into the lumen of the inner conduit of the nebulizer body.
- the inspiration valve of the cover is facing and in fluid communication with the air inlet located at the upper end of the axially-arranged inner conduit.
- the nebulization chamber is located above the liquid tank.
- the cover comprises a grip portion for allowing an easy removal, i.e. detachment, of the cover from the nebulizer body.
- the cover comprises a grip portion, such as a textured area, preferably arranged on its outer wall, that allows the user to easily decouple the cover from the nebulizer body.
- the cover forms a roof or top of the nebulizer body.
- the liquid tank has a cup-like shape.
- the aerosol-generating system comprises a nozzle element for accelerating the air delivered by a source of compressed air, thereby creating a Venturi or suction effect pulling some liquid solution out of the liquid tank.
- the aerosol-generating system comprises deflector element for atomizing the liquid thereby creating a mist containing liquid droplets.
- the cover is detachably secured to the nebulizer body, i.e. press-fitted, snap-fitted or similarly detachably affixable to the nebulizer body.
- the nebulizer body, the reservoir and/or the cover are made of polymer, such as PP, PC, ABS, PA, PSU or any other suitable plastic material.
- the respiratory interface comprises a proximal end connected to the nebulizer body and a distal end that is free.
- the distal end of the respiratory interface comprises the exit orifice of the inner passage.
- the reservoir secured to the nebulizer body by one or several pin elements, such as picots, dents or the like.
- the one or several pin elements are arranged on the outer surface of the reservoir.
- the peripheral wall of the nebulizer body comprise one or several lodgings, such as bores, blind-holes, grooves or the like.
- the one or several pin elements of the reservoir cooperate with the one or several lodgings of the nebulizer body for securing the reservoir to the nebulizer body, i.e. maintaining those parts integral together.
- the one or several pin elements of the reservoir fit(s) into the one or several lodgings of the nebulizer body thereby ensuring a good positioning of the reservoir with respect to the nebulizer body and improving the securing of those parts together, when they are snap-fitted or the like.
- the one or several lodgings of the nebulizer body are bore(s) traversing the peripheral wall of the nebulizer body.
- the nebulizer body comprises a neck portion allowing a user to seize the nebulizer in one hand by gripping said neck portion, i.e. a region of the body having a restricted section.

A preferred embodiment of a nebulizer according to the present invention is shown in the enclosed Figures, among which:
- Figure 1 represents an exploded view of an embodiment of a nebulizer according to the present invention,
- Figure 2 is an enlarged view of the cover of Figure 1,
- Figure 3 is a longitudinal sectional view of the nebulizer of Figure 1,
- Figure 4 is an elevation view of the reservoir of the nebulizer of Figure 1,
- Figures 5a-5c illustrate the assembling of the reservoir to the nebulizer body of the nebulizer of Figure 1,
- Figures 6a-6c illustrate the assembling of the cover to the nebulizer body of the assembly of Figure 5c, and
- Figures 7a-7b illustrate the connecting of a respiratory interface, e.g. a mouthpiece, to the nebulizer body of the assembly of Figures 6c.

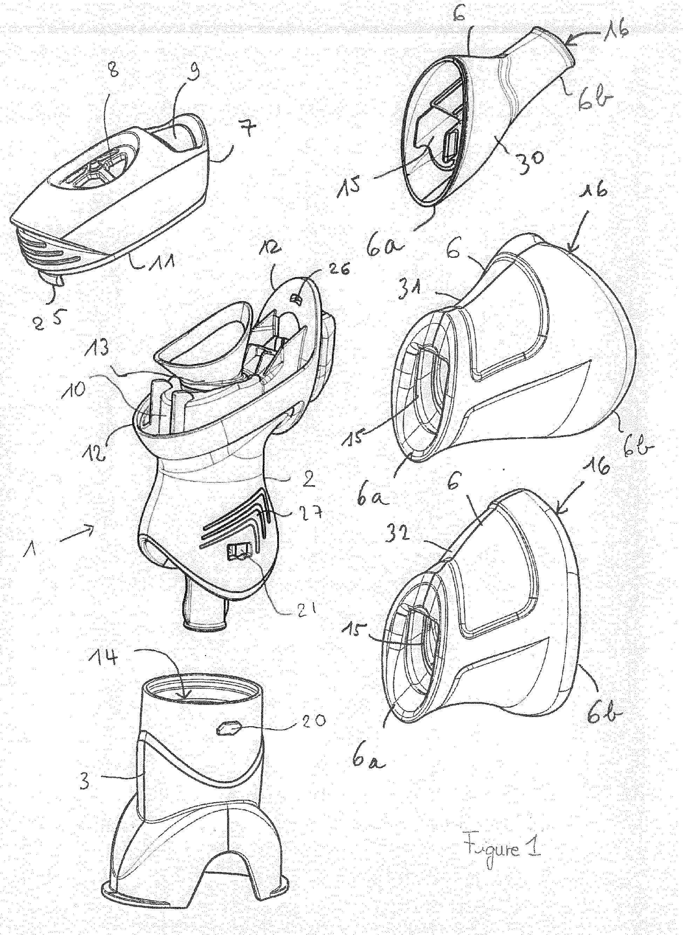

Figure 1 represents an exploded view an embodiment of a nebulizer 1 according to the present invention designed for aerosolizing a liquid, such as a drug useable in aerosoltherapy, which is made of 4 main sub-parts or sub-units detachably fixed together, namely:
- a reservoir 3 forming the lower part of the nebulizer 1, and which is designed for receiving and containing a liquid to be aerosolized, in particular a drug-containing solution,
- a nebulizer body 2 comprising an aerosol-generating system 4, 23, 24 for generating an aerosol, i.e. a mist, and a nebulization chamber 5 for containing the aerosol, i.e. the mist, generated by the aerosol-generating system 4, 23, 24,
- a detachable cover 7 comprising a double-valve system 8, 9, i.e. comprising two valves 8, 9, which is secured to the nebulizer body 2, and
- a respiratory interface 6 for delivering the drug-containing mist to a patient.

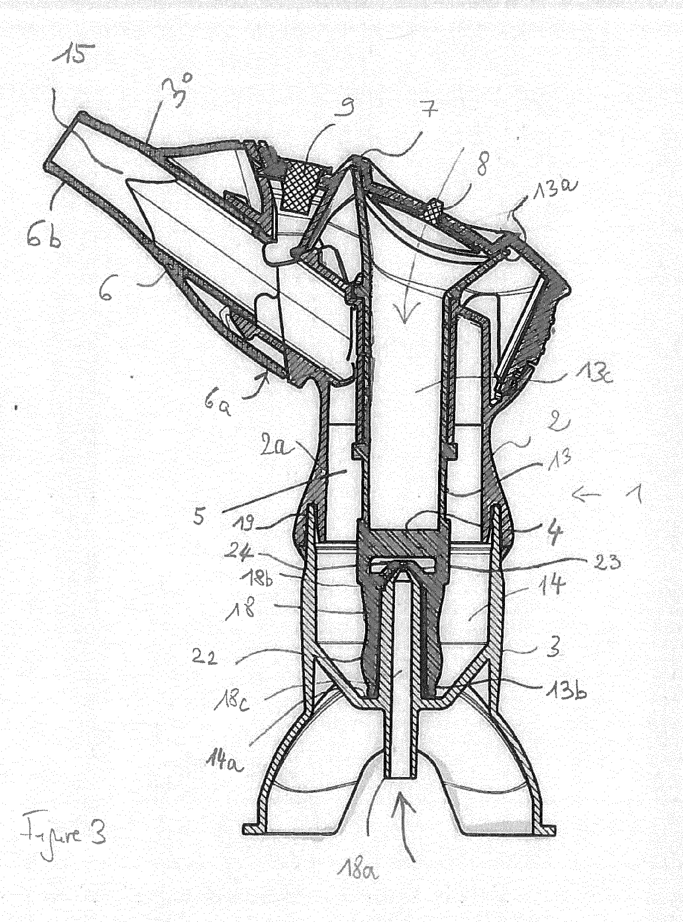

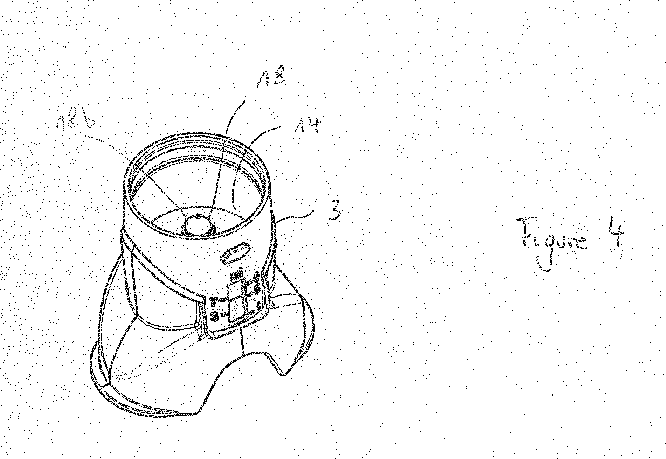

As shown in Figures 1, 3 and 4, the reservoir 3 comprises a liquid tank 14, also called liquid vessel or compartment, for receiving the liquid to be aerosolized, and further an inner conduit 18, as shown in Fig. 3, arranged so as to project axially and upwardly into the liquid tank 14 of the reservoir 3. Preferably, the tank 14 of the reservoir 3 has a "cup-like" shape.

Furthermore, the inner conduit 18 is arranged at the center of the liquid tank 14 of the reservoir 3. The role of the inner conduit 18 is to convey a pressurized gas delivered by a gas source (not shown), such as compressed air, fluidly connected to the upstream end 18a of the inner conduit 18. The gas flow travels into the lumen or inner passage 18c of the inner conduit 18 and is delivered by the downstream end 18b of the inner conduit 18 for creating the mist or aerosol as explained below.

The reservoir 3 is detachably attached to the nebulizer body 2, for instance they are snap-fitted or press-fitted together, or similarly detachably affixable. Preferably, the reservoir 3 is adapted for repeated attachment/detachment.

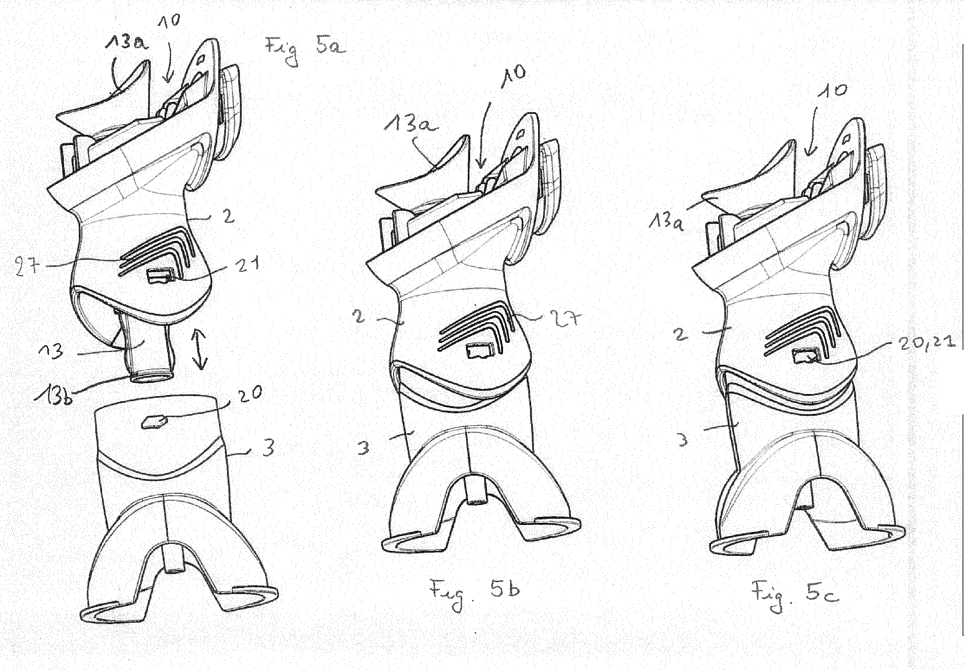

In the embodiment of Figure 1 and as shown in Figures 5a-5c, when the reservoir 3 is assembled by means of a snap-fit connection or similar (19 in Figure 3) to the nebulizer body 2, one (or several) pin element(s) 20, such as picots or dents, arranged on the outer surface of the reservoir 3 fit(s) into, i.e. is (are) received into, one (or several) lodging(s) 21, such as bores, blind-holes or grooves, arranged in the peripheral wall of the nebulizer body 2, i.e. bore(s) traversing the peripheral wall of the nebulizer body 2. This ensures a good positioning of the reservoir 3 with respect to the nebulizer body 2 as well as an improved fixation of those parts together, when they are snap-fitted or the like.

The nebulizer body 2 comprises, as shown in Figure 3:
- an aerosol-generating system 4 for generating an aerosol, i.e. a mist,
- a nebulization chamber 5 for containing the aerosol, i.e. the mist, generated by the aerosol-generating system 4, and
- an axially-arranged inner conduit 13 comprising an air inlet at its upper end 13a, an air outlet 13b at its lower end 13b and an internal passage 13c arranged in-between for conveying ambient air from the air inlet towards the air outlet.

Further, the nebulizer body 2 forming the main part of the nebulizer 1 comprises a top opening 10 that is closed by an upper cover 7 forming the "top" or "roof" of the nebulizer body 2 as shown in Figure 1.

Preferably, at least a part of the nebulization chamber 5 is positioned between the peripheral wall 2a of the nebulizer body 2 and the axially-arranged inner conduit 13, and further located above the liquid tank 14.

As shown in Figure 3, when the reservoir 3 is assembled to the nebulizer body 2, the downstream end 18b of the inner conduit 18 is inserted into the internal passage 13c of the lower end 13b of the axially-arranged inner conduit 13, i.e. the downstream end 18b of the inner conduit 18 passes through the air outlet 13b of the the axially-arranged inner conduit 13 of the nebulizer body 2.

In other words, the lower end 13b of the axially-arranged inner conduit 13 of the nebulizer body 2 forms a sleeve around the downstream end 18b of the inner conduit 18 of the reservoir 3.

The lower end 13b of the axially-arranged inner conduit 13 of the nebulizer body 2 further projects into the liquid tank 14 of the reservoir 3 and terminates close to the bottom 14a of the liquid tank 14 as shown in Figure 3, preferably at a distance of less than about 2 mm.

In the aim of allowing a circulation of liquid between them, a spacing 22 is kept, on the one hand, between the extremity of the lower end 13b of the axially-arranged inner conduit 13 and the bottom 14a of the liquid tank 14 and, on the second hand, between the inner wall of the lower end 13b of the axially-arranged inner conduit 13 and the outer wall of the downstream end 18b of the inner conduit 18 of the reservoir 3. Preferably, the distance between the inner wall of the lower end 13b of the axially-arranged inner conduit 13 and the outer wall of the downstream end 18b of the inner conduit 18 of the reservoir 3 is of less than about 1 mm.

Indeed, for generating the aerosol mist, the liquid contained into the tank 14 of the reservoir 3 should be able to circulate in said spacing 22 for reaching the aerosol-generating system 4, 23, 24, as explained below.

The aerosol-generating system 4, 23, 24 is arranged in the lumen 13c of the inner conduit 13 of the nebulizer body 2 and comprises, as shown in Figure 3 :
- a nozzle element 23 with a small orifice 24 forming a flow restriction, for instance a restriction orifice 24 having a diameter or dimensions of between about 1 mm to 5 mm. The orifice 24 can have a circular section, an ellipse section or any other suitable shape. Preferably, the nozzle element 23 is axially arranged into lumen 13c.
- and a deflector element 4 facing the small orifice 24 of the nozzle element 23, such as a little wall, blade, pin or similar that is arranged into lumen 13c, preferably radially-positioned into lumen 13c.

Such an aerosol-generating system 4, 23, 24 can generate the aerosol mist thanks to a Venturi effect. This principle is well-known in the art. In few words, pressurized air is conveyed and delivered by by the inner conduit 18 of the reservoir 3, at a high speed and a constant flowrate. This creates a suction effect that moves the liquid solution out of the tank 14. The liquid solution travels in the spacing 22, i.e. it is upwardly pulled by the suction effect, and is then delivered by the small orifice 24 of the nozzle element 23 toward the deflector element 4. When the liquid enters into contact with the deflector element 4, a mist of liquid droplets is created by the impact of the liquid on the deflector element 4. The smaller droplets create the desired aerosol mist that is recovered into the nebulization chamber 5, whereas the larger and/or heavier droplets return to the liquid tank 14.

The aerosol mist can thereafter be inhaled by the patient via a respiratory interface 6.

Indeed, a respiratory interface 6, also called "patient interface", is fixed to the nebulizer body 2 and is in fluid communication with the nebulization chamber 5 so that the aerosol created in the nebulization chamber 5 can be delivered to a patient by said respiratory interface 6 as illustrated in Figure 3.

As shown in Figure 1, the respiratory interface 6 can be a mouthpiece 30, or a mouth or facial respiratory mask 31, 32, or similar. The choice of the most suitable interface 6 depends on the type of medication to be taken and on the type of patients i.e. the facial mask is suitable for non collaborative pediatric patients, while the mouthpiece and mouth mask are better for adults.

In the embodiment of Figures 1, 3 and 7a-7b, the respiratory interface 6 is a mouthpiece 30 with an inner passage 15, that is designed so that the patient takes it into his/her mouth for inhaling the drug-containing mist. Actually, the mouthpiece 30 is traversed by an inner passage 15 that is in fluid communication with the nebulization chamber 5.

Generally speaking, the respiratory interface 6 comprises a proximal end 6a connected to the nebulizer body 2 and a distal end 6b that is free and which comprises the exit orifice 16 of the inner passage 15.

In the case of a mouthpiece 30, the distal end 6b is further designed so as to form a "beak-shape nozzle" that the patient takes in mouth, whereas in the case of a mouth or facial respiratory mask 31, 32, the distal end 6b is further designed so as to form an enclosure that is large enough for receiving at least a part of the patient's nose or nose and mouth, through the exit orifice 16.

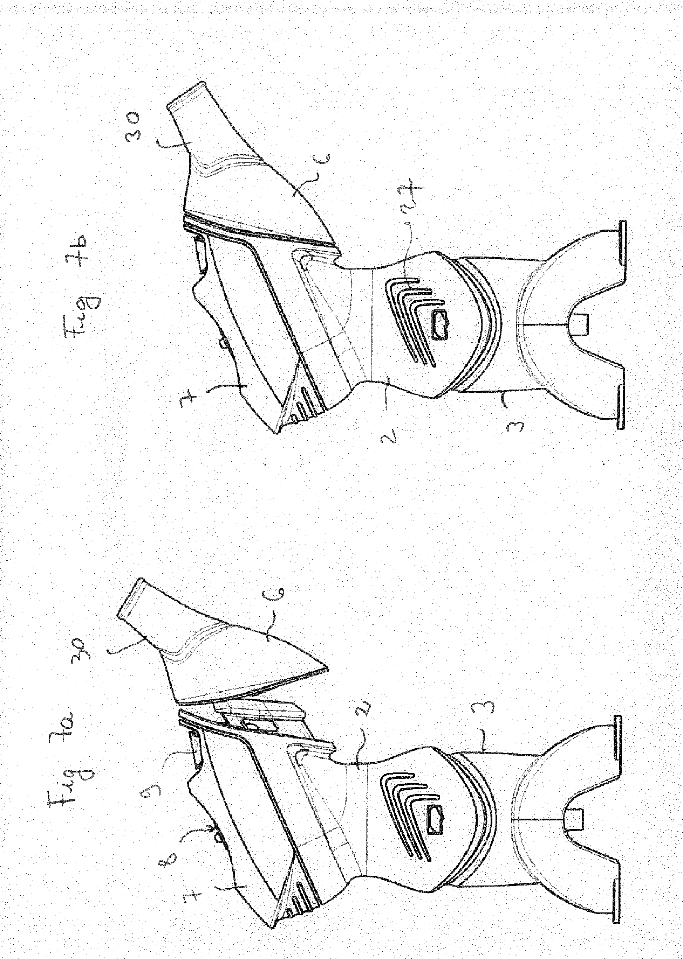

Figures 7a-7b show the attachment of a mouthpiece 30 to the nebulizer body 2 of the nebulizer 1 of Figure 1. However, the attaching of a mouth or facial respiratory mask 31, 32 can be operated similarly.

Furthermore, the nebulizer 1 further comprises a detachable cover 7 that forms the "top" of the nebulizer 1 as forming the ceiling of the nebulizer body 2 as illustrated in Figures 1, 2 and 3.

More precisely, the cover 7 comprises a lower peripheral border 11 cooperating with an upper peripheral border 12 delimiting the top opening 10 of the nebulizer body 2.

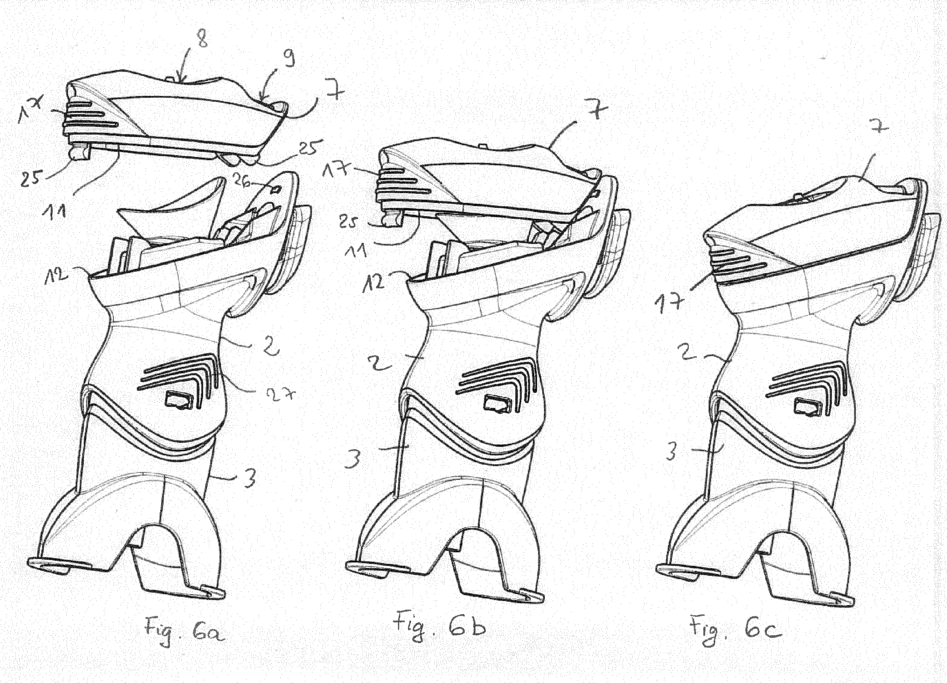

For maintaining the cover 7 detachably attached to the nebulizer body 2, the cover 7 can, for instance, be snap-fitted or similar to the nebulizer body 2 so that the lower peripheral border 11 of the cover 7 cooperates with the upper peripheral border 12 of the top opening 10 of the nebulizer body 2. As shown in Figures 1, 2 and 6a-6c, connecting structures 25, 26, such as pin(s), finger(s), claw(s), clamp(s) 25 or the like and corresponding lodging(s) 26, groove(s) or the like, are arranged on the cover 7 and/or the nebulizer body 2 and cooperate together for allowing a tight and integral fixing of the cover 7 to the nebulizer body 2.

Preferably, the top cover 7 further comprises a grip portion 17 arranged in its outer wall that allows the user, e.g. the patient, to easily remove the cover 7 when attached to the nebulizer body 2, for instance for cleaning the nebulization chamber 5. The grip portion 17 can comprise a textured area, i.e. a not-smooth surface, allowing a good grip to the user, e.g. a textured area comprising "ridges" and "valleys".

Furthermore, similar grip portions 27 are also arranged in the peripheral wall 2a of the nebulizer body 2 for improving the hand grip of the nebulizer 1 by the patient.

Preferably, the cover 7 is adapted for repeated attachment/detachment.

As shown in Fig. 1 and 5-7, the nebulizer body 2 also comprises a neck portion i.e. a region of the body having a restricted section. This allows a user to seize the nebulizer 1 in one hand in gripping said neck portion. This is an advantage for users having small hands, such as children. Indeed, the nebulizer 1 of the present invention is a portative device that can be carried in hand by a patient.

Further, the cover 7 of the nebulizer 1 comprises a valve system 8, 9 comprising a pair of valves, i.e.
- a one-way inspiration valve 8 allowing ambient air entering into the nebulization chamber 5 via the axially-arranged inner conduit 13 of the nebulizer body 2, during the inspiration phases of the patient, and
- a one-way expiration valve 9 allowing gas exiting the nebulization chamber 5, during the expiration phases of the patient, especially expired gases that contains CO₂.

Preferably, the one-way expiration valve 9 is located between the one-way inspiration valve 8 and the respiratory interface 6, when the cover 7 is integrally fixed to the nebulizer body 2.

In other words, the inspiration valve 8 is located on the cover 7 facing and in fluid communication with the air inlet located at the upper end 13a of the axially-arranged inner conduit 13, and opens only during inspiration phases of the patient by action of the negative pressure generated by the patient's inspiration, i.e. during inspiration phases.

The inspiration valve 8 provides an additional flow of ambient air that enters into the nebulization chamber 5 thereby boosting the nebulization (due to a "double" Venturi effect) and significantly increasing the amount of aerosol mist formed into said nebulizing chamber 5, and further thereby increasing the nebulization rate of the liquid drug, i.e. the solution containing the medication.

In other words, the on-way inspiration valve 8 allows air to only travel from the outside to the inside of the nebulizer 1, i.e. to enter into the nebulization chamber 5 via the axially-arranged inner conduit 13.

Further, the expiration valve 9 is located next to the inspiration valve 8 on the detachable cover 7, but closer to the mouthpiece 6 than the inspiration valve 8.

Said expiration valve 9 opens by action of the CO₂-containing gases exhaled by the patient that create an overpressure (i.e. pressure > 1 bar) in the respiratory interface 6 and at the inlet of the nebulization chamber 5, while the patient is exhaling gases. Said overpressure allows gases to be vented from the nebulizer 1, through the expiration valve 9, when the patient exhales into the respiratory interface 6. In other words, the on-way expiration valve 9 allows said CO₂-containing gas to only travel from the inside to the outside of the nebulizer 1, i.e. to be vented to the atmosphere.

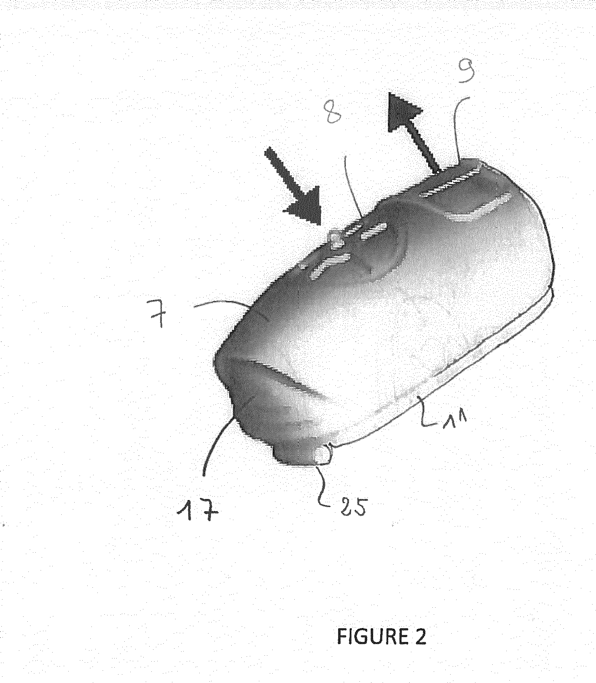

With a nebulizer 1 according to the present invention, the patient does not remove the respiratory interface 6, such as the mouthpiece 30 out of his/her mouth, before starting to expire CO₂-containing gases. In other words, thanks to the nebulizer 1 according to the present invention, the patient can execute his/her aerosoltherapy by both inhaling and exhaling gases through the respiratory interface 6 and the cover 7 (see arrows in Fig. 2), the amount of aerosolized drug wasted in the environment is considerably reduced, compared with prior art nebulizers, as the exit of expired gases is controlled by the one-way expiration valve 9, i.e. there is no permanent opening for exhaled air.

Furthermore, it is advantageous to arranged both valves 8, 9 on the detachable cover 7, rather than on the respiratory interface 6 because it reduces the number of expiration valves that otherwise would have been necessary for using the nebulizer 1 with different kinds of respiratory interfaces 6, i.e. mouthpiece 30, mouth and facial masks 31, 32, each equipped with an expiration valve.

All the components of the nebulizer 1, i.e. cover 7, nebulizer body 2, are preferably made of plastic material(s), such as PP, PC, ABS, PA, PSU or similar materials, while the masks are made of a soft elastomeric material, such as silicone or the like.

The nebulizer 1 of the present invention is usable in combination with a source of gas, such as compressed air, for nebulizing a drug-containing solution and thereby obtaining a mist containing droplets of medication having suitable sizes so as to be able to reach the lower airways of the patient that has to inhale said mist in the course of his/her aerosoltherapy.

## Claims

1. Nebulizer (1) for aerosolizing a liquid consisting in 4 sub-units (2, 3, 6, 7) detachably secured together, said 4 sub-units (2, 3, 6, 7) being :
- a nebulizer body (2) comprising an aerosol-generating system (4; 23, 24) for generating an aerosol and a nebulization chamber (5) for containing the aerosol, and further comprises a first axially-arranged inner conduit (13) comprising a lumen (13c) and a lower end (13b) projecting into the liquid tank (14) of the reservoir (3), the lower end (13b) of the first inner conduit (13) terminating close to the bottom (14a) of the liquid tank (14),
- a reservoir (3) comprises a liquid tank (14) for receiving a liquid to be aerosolized, the reservoir (3) being detachably fixed to the nebulizer body (2), and further comprises a second inner conduit (18) projecting axially and upwardly into the liquid tank (14), the first inner conduit (13) of the nebulizer body (2) being arranged as a sleeve around a downstream end (18b) of the second inner conduit (18) of the reservoir (3) and being spaced from the downstream end (18b) of the second inner conduit (18) of the reservoir (3) by a spacing (22),
- a detachable cover (7) fixed to the nebulizer body (2) and forming the top of the nebulization chamber (5), and
- a respiratory interface (6) detachably fixed to and in fluid communication with the nebulization chamber (5),
**characterized in that** the aerosol-generating system (4; 23, 24) of the nebulizer body (2) comprises a nozzle element (23) comprising an orifice (24) and a deflector element (4) facing the orifice (24) of the nozzle element (23), said nozzle element (23) and said deflector element (4) being arranged into the lumen (13c) of the first inner conduit (13), and
the detachable cover (7) comprises at least two valves (8,9).

2. Nebulizer according to the preceding Claim, **characterized in that** the cover (7) comprises a one-way inspiration valve (8) allowing air entering into the nebulization chamber (5) and a one-way expiration valve (9) allowing gas exiting the nebulization chamber (5).

3. Nebulizer according to any one of the preceding Claims, **characterized in that** the nebulizer body (2) is sandwiched between the cover (7) and the reservoir (3).

4. Nebulizer according to Claim 2, **characterized in that** the one-way expiration valve (9) is located between the one-way inspiration valve (8) and the respiratory interface (6), when the cover (7) is fixed to the nebulizer body (2).

5. Nebulizer according to any one of the preceding Claims, **characterized in that** the respiratory interface (6) comprises a mouthpiece (30), a mouth mask (31) or a facial mask (32).

6. Nebulizer according to Claim 2, **characterized in that** the inspiration valve (8) of the cover (7) is facing and in fluid communication with the air inlet located at the upper end (13a) of the axially-arranged inner conduit (13).

7. Nebulizer according to any one of the preceding Claims, **characterized in that** the nebulization chamber (5) is located above the liquid tank (14).

8. Nebulizer according to any one of the preceding Claims, **characterized in that** the cover (7) comprises a grip portion (17).

9. Nebulizer according to Claim 1, **characterized in that** at least one pin elements (20) arranged on an outer surface of the reservoir (3) fits into at least one lodging (21) comprised in a peripheral wall of the nebulizer body (2) thereby assembling the reservoir (3) to the nebulizer body (2) by means of a snap-fit connection (19).

10. Nebulizer according to Claims 1 or 9, **characterized in that** several pin elements (20) arranged on the outer surface of the reservoir (3) fit into several lodgings (21) arranged in the peripheral wall of the nebulizer body (2).

11. Nebulizer according to Claims 9 or 10, **characterized in that** said one or several lodgings (21) comprised in the peripheral wall of the nebulizer body (2) are one or several bores traversing the peripheral wall of the nebulizer body (2).

12. Nebulizer according to Claim 1, **characterized in that** at least a part of the nebulization chamber (5) is positioned between the peripheral wall (2a) of the nebulizer body (2) and the axially-arranged inner conduit (13), and further located above the liquid tank (14).

13. Nebulizer according to Claim 1, **characterized in that** the lower end (13b) of the inner conduit (13) terminating at a distance of less than about 2 mm from the bottom (14a) of the liquid tank (14).

14. Nebulizer according to Claim 1, **characterized in that** the nebulizer body comprises a neck portion allowing a user to seize the nebulizer in one hand by gripping said neck portion.

15. Nebulizer according to Claim 14, **characterized in that** the nebulizer body comprises a region having a restricted section forming the neck portion.

## Patentansprüche

1. Vernebler (1) zum Aerosolisieren einer Flüssigkeit, die aus 4 Untereinheiten (2, 3, 6, 7) besteht, die abnehmbar aneinander befestigt sind, wobei die 4 Untereinheiten (2, 3, 6, 7) sind:
- ein Verneblerkörper (2), der ein Aerosol erzeugendes System (4; 23, 24) zum Erzeugen eines Aerosols und eine Vernebelungskammer (5) zum Enthalten des Aerosols umfasst, und weiter eine erste axial angeordnete innere Leitung (13) umfasst, die ein Lumen (13c) und ein unteres Ende (13b) umfasst, das in den Flüssigkeitstank (14) des Behälters (3) ragt, wobei das untere Ende (13b) der ersten inneren Leitung (13) nahe dem Boden (14a) des Flüssigkeitstanks (14) endet,
- ein Behälter (3), der einen Flüssigkeitstank (14) zum Aufnehmen einer zu aerosolisierenden Flüssigkeit umfasst, wobei der Behälter (3) abnehmbar an dem Verneblerkörper (2) befestigt ist, und weiter eine zweite innere Leitung (18) umfasst, die axial und aufwärts in den Flüssigkeitstank (14) ragt, wobei die erste innere Leitung (13) der Verneblerkörpers (2) als Muffe um ein stromabwärtiges Ende (18b) der zweiten inneren Leitung (18) des Behälters (3) angeordnet ist, und von dem stromabwärtigen Ende (18b) der zweiten inneren Leitung (18) des Behälters (3) durch einen Abstandhalter (22) beabstandet ist,
- eine abnehmbare Abdeckung (7), die an dem Verneblerkörper (2) befestigt ist, und die Oberseite der Vernebelungskammer (5) bildet, und
- eine Atmungsschnittstelle (6), die abnehmbar an der Verneblerkammer (5) befestigt und damit in Fluidverbindung ist,
**dadurch gekennzeichnet, dass** das Aerosol erzeugende System (4; 23, 24) des Verneblerkörpers (2) ein Düsenelement (23) umfasst, das eine Öffnung (24) und ein Abweiserelement (4) umfasst, das der Öffnung (24) des Düsenelements (23) zugewandt ist, wobei das Düsenelement (23) und das Abweiserelement (4) in dem Lumen (13c) der ersten inneren Leitung (13) angeordnet sind, und
die abnehmbare Abdeckung (7) mindestens zwei Ventile (8, 9) umfasst.

2. Vernebler nach dem vorstehenden Anspruch, **dadurch gekennzeichnet, dass** die Abdeckung (7) ein Einweg-Einatmungsventil (8) umfasst, das es Luft ermöglicht, in die Vernebelungskammer (5) einzutreten, und ein Einweg-Ausatmungsventil (9), das es Gas ermöglicht, aus der Vernebelungskammer (5) auszutreten.

3. Vernebler nach einem der vorstehenden Ansprüche, **dadurch gekennzeichnet, dass** der Verneblerkörper (2) zwischen der Abdeckung (7) und dem Behälter (3) sandwichartig angeordnet ist.

4. Vernebler nach Anspruch 2, **dadurch gekennzeichnet, dass** sich das Einweg-Ausatmungsventil (9) zwischen dem Einweg-Einatmungsventil (8) und der Atmungsschnittstelle (6) befindet, wenn die Abdeckung (7) an dem Verneblerkörper (2) befestigt ist.

5. Vernebler nach einem der vorstehenden Ansprüche, **dadurch gekennzeichnet, dass** die Atmungsschnittstelle (6) ein Mundstück (30), eine Mundmaske (31) oder eine Gesichtsmaske (32) umfasst.

6. Vernebler nach Anspruch 2, **dadurch gekennzeichnet, dass** das Einatmungsventil (8) der Abdeckung (7) dem Lufteinlass, der sich am oberen Ende (13a) der axial angeordneten inneren Leitung (13) befindet, zugewandt ist und damit in Fluidverbindung ist.

7. Vernebler nach einem der vorstehenden Ansprüche, **dadurch gekennzeichnet, dass** sich die Vernebelungskammer (5) oberhalb des Flüssigkeitstank (14) befindet.

8. Vernebler nach einem der vorstehenden Ansprüche, **dadurch gekennzeichnet, dass** die Abdeckung (7) einen Greifabschnitt (17) umfasst.

9. Vernebler nach Anspruch 1, **dadurch gekennzeichnet, dass** mindestens ein Achsenelemente (20), das an einer äußeren Oberfläche des Behälters (3) angeordnet ist, in mindestens eine Unterbringung (21) passt, die in einer umlaufenden Wand des Verneblerkörpers (2) umfasst ist, wodurch der Behälter (3) mit dem Verneblerkörper (2) anhand einer Schnappsitzverbindung (19) zusammengesetzt wird.

10. Vernebler nach den Ansprüchen 1 oder 9, **dadurch gekennzeichnet, dass** verschiedene Achsenelemente (20), die an einer äußeren Oberfläche des Behälters (3) angeordnet sind, in verschiedene Unterbringungen (21) passen, die in der umlaufenden Wand des Verneblerkörpers (2) angeordnet sind.

11. Vernebler nach den Ansprüchen 9 oder 10, **dadurch gekennzeichnet, dass** die eine oder verschiedene Unterbringungen (21), die in der umlaufenden Wand des Verneblerkörpers (2) umfasst sind, eine oder verschiedene Bohrungen sind, die die umlaufende Wand des Verneblerkörpers (2) durchqueren.

12. Vernebler nach Anspruch 1, **dadurch gekennzeichnet, dass** mindestens ein Teil der Vernebelungskammer (5) zwischen der umlaufenden Wand (2a) des Verneblerkörpers (2) und der axial angeordneten inneren Leitung (13) positioniert ist, und sich weiter oberhalb des Flüssigkeitstanks (14) befindet.

13. Vernebler nach Anspruch 1, **dadurch gekennzeichnet, dass** das untere Ende (13b) der inneren Leitung (13) in einem Abstand von weniger als etwa 2 mm von dem Boden (14a) des Flüssigkeitstanks (14) endend.

14. Vernebler nach Anspruch 1, **dadurch gekennzeichnet, dass** der Verneblerkörper einen Halsabschnitt umfasst, der es einem Nutzer ermöglicht, den Vernebler mit einer Hand durch Greifen des Halsabschnitts zu fassen.

15. Vernebler nach Anspruch 14, **dadurch gekennzeichnet, dass** der Verneblerkörper eine Region umfasst, die einen beschränkten Bereich aufweist, der den Halsabschnitt bildet.

## Revendications

1. Atomiseur (1) pour pulvériser un liquide consistant en 4 sous-unités (2, 3, 6, 7) fixées l'une à l'autre de manière détachable, lesdites 4 sous-unités (2, 3, 6, 7) étant :
- un corps d'atomiseur (2) comprenant un système générateur d'aérosol (4 ; 23, 24) pour générer un aérosol et une chambre d'atomisation (5) pour contenir l'aérosol, et comprend en outre un premier conduit interne axialement agencé (13) comprenant une lumière (13c) et une extrémité inférieure (13b) faisant saillie dans la retenue de liquide (14) du réservoir (3), l'extrémité inférieure (13b) du premier conduit interne (13) se terminant à proximité du fond (14a) de la retenue de liquide (14),
- un réservoir (3) comprend une retenue de liquide (14) pour recevoir un liquide à pulvériser, le réservoir (3) étant fixé de manière détachable au corps d'atomiseur (2), et comprend en outre un second conduit interne (18) faisant saillie axialement et vers le haut dans la retenue de liquide (14), le premier conduit interne (13) du corps d'atomiseur (2) étant agencé sous la forme d'un manchon autour d'une extrémité aval (18b) du second conduit interne (18) du réservoir (3) et étant espacé de l'extrémité aval (18b) du second conduit interne (18) du réservoir (3) par un espacement (22),
- un couvercle détachable (7) fixé au corps d'atomiseur (2) et formant le sommet de la chambre d'atomisation (5), et
- une interface respiratoire (6) fixée de manière détachable à et en communication fluidique avec la chambre d'atomisation (5),
**caractérisé en ce que** le système générateur d'aérosol (4 ; 23, 24) du corps d'atomiseur (2) comprend un élément formant buse (23) comprenant un orifice (24) et un élément déflecteur (4) en regard de l'orifice (24) de l'élément formant buse (23), ledit élément formant buse (23) et ledit élément déflecteur (4) étant agencés dans la lumière (13c) du premier conduit interne (13), et
le couvercle détachable (7) comprend au moins deux valves (8, 9).

2. Atomiseur selon la revendication précédente, **caractérisé en ce que** le couvercle (7) comprend une valve d'inspiration à voie unique (8) permettant à l'air de pénétrer dans la chambre d'atomisation (5) et une valve d'expiration à voie unique (9) permettant au gaz de quitter la chambre d'atomisation (5).

3. Atomiseur selon l'une quelconque des revendications précédentes, **caractérisé en ce que** le corps d'atomiseur (2) est pris en sandwich entre le couvercle (7) et le réservoir (3).

4. Atomiseur selon la revendication 2, **caractérisé en ce que** la valve d'expiration à voie unique (9) est située entre la valve d'inspiration à voie unique (8) et l'interface respiratoire (6), lorsque le couvercle (7) est fixé au corps d'atomiseur (2).

5. Atomiseur selon l'une quelconque des revendications précédentes, **caractérisé en ce que** l'interface respiratoire (6) comprend une pièce buccale (30), un masque buccal (31) ou un masque facial (32).

6. Atomiseur selon la revendication 2, **caractérisé en ce que** la valve d'inspiration (8) du couvercle (7) est en regard de et en communication fluidique avec l'entrée d'air située à l'extrémité supérieure (13a) du conduit interne axialement agencé (13).

7. Atomiseur selon l'une quelconque des revendications précédentes, **caractérisé en ce que** la chambre d'atomisation (5) est située au-dessus de la retenue de liquide (14).

8. Atomiseur selon l'une quelconque des revendications précédentes, **caractérisé en ce que** le couvercle (7) comprend une portion de préhension (17).

9. Atomiseur selon la revendication 1, **caractérisé en ce qu'**au moins un éléments formant broche (20) agencé sur une surface externe du réservoir (3) s'ajuste dans au moins un logement (21) compris dans une paroi périphérique du corps d'atomiseur (2), assemblant de la sorte le réservoir (3) au corps d'atomiseur (2) au moyen d'un encliquetage (19).

10. Atomiseur selon les revendications 1 ou 9, **caractérisé en ce que** plusieurs éléments formant broche (20) agencés sur la surface externe du réservoir (3) s'ajustent dans plusieurs logements (21) agencés dans la paroi périphérique du corps d'atomiseur (2).

11. Atomiseur selon les revendications 9 ou 10, **caractérisé en ce que** lesdits un ou plusieurs logements (21) compris dans la paroi périphérique du corps d'atomiseur (2) sont un ou plusieurs alésages traversant la paroi périphérique du corps d'atomiseur (2).

12. Atomiseur selon la revendication 1, **caractérisé en ce qu'**au moins une partie de la chambre d'atomisation (5) est positionnée entre la paroi périphérique (2a) du corps d'atomiseur (2) et le conduit interne axialement agencé (13), et en outre située au-dessus de la retenue de liquide (14).

13. Atomiseur selon la revendication 1, **caractérisé en ce que** l'extrémité inférieure (13b) du conduit interne (13) se terminant à une distance de moins d'environ 2 mm du fond (14a) de la retenue de liquide (14).

14. Atomiseur selon la revendication 1, **caractérisé en ce que** le corps d'atomiseur comprend une portion de col permettant à un utilisateur de saisir l'atomiseur d'une main en saisissant ladite portion de col.

15. Atomiseur selon la revendication 14, **caractérisé en ce que** le corps d'atomiseur comprend une région ayant une section restreinte formant la portion de col.
